# EUROPÄISCHE PATENTSCHRIFT

(11) **EP 3 099 671 B1**
(45) Veröffentlichungstag und Bekanntmachung des Hinweises auf die Patenterteilung: **27.12.2017**
(21) Anmeldenummer: 15701198.2
(22) Anmeldetag: 23.01.2015
(51) Int. Cl.: C07D 307/93, C07C 209/58, C07D 209/48, C07F 7/18

(54) **VERFAHREN ZUR HERSTELLUNG VON 1-INDANOLEN UND 1-INDANAMINEN**
METHOD FOR THE PRODUCTION OF 1-INDANOLES AND 1-INDANAMINES
PROCÉDÉ DE FABRICATION DE 1-INDANOLES ET 1-INDANAMINES

(30) Priorität: 28.01.2014 EP 14152790
(43) Veröffentlichungstag der Anmeldung: 07.12.2016
(73) Patentinhaber: Bayer CropScience Aktiengesellschaft, 40789 Monheim am Rhein (DE)
(72) Erfinder: FORD, Mark James, 65207 Wiesbaden-Breckenheim (DE); VORS, Jean-Pierre, 69110 Sainte Foy les Lyon (FR); BAUDOIN, Olivier, 68100 Mulhouse (FR); JANODY, Simon, 69730 Genay (FR)
(74) Vertreter: BIP Patents
(86) Internationale Anmeldenummer: PCT/EP2015/051344
(87) Internationale Veröffentlichungsnummer: WO 2015/113903

(56) Entgegenhaltungen:
- J. A. PINCOCK ET AL: "The Photochemistry of Conformationally Rigid Benzylic Esters: 2,2-Dimethyl-1-indanyl Acetates and Pivalates", THE JOURNAL OF ORGANIC CHEMISTRY, Bd. 60, Nr. 13, 1. Juni 1995 (1995-06-01), Seiten 4067-4076, XP055123358, ISSN: 0022-3263, DOI: 10.1021/jo00118a024 in der Anmeldung erwähnt
- HUI FANG ET AL: "Rapid Catalyst Screening by a Continuous-Flow Microreactor Interfaced with Ultra-High-Pressure Liquid Chromatography", THE JOURNAL OF ORGANIC CHEMISTRY, Bd. 75, Nr. 16, 20. August 2010 (2010-08-20), Seiten 5619-5626, XP055123361, ISSN: 0022-3263, DOI: 10.1021/jo100981e in der Anmeldung erwähnt
- CATHLEEN PIERRE ET AL: "Synthesis of Polycyclic Molecules by Double C(sp 2 )-H/C(sp 3 )-H Arylations with a Single Palladium Catalyst", ORGANIC LETTERS, Bd. 13, Nr. 7, 1. April 2011 (2011-04-01), Seiten 1816-1819, XP055123277, ISSN: 1523-7060, DOI: 10.1021/ol200329e in der Anmeldung erwähnt
- NICOLAS MARTIN ET AL: "Diastereo- and Enantioselective Intramolecular C(sp3)-H Arylation for the Synthesis of Fused Cyclopentanes", CHEMISTRY - A EUROPEAN JOURNAL, Bd. 18, Nr. 15, 10. April 2012 (2012-04-10), Seiten 4480-4484, XP055123282, ISSN: 0947-6539, DOI: 10.1002/chem.201200018 in der Anmeldung erwähnt

## Beschreibung

Die Erfindung betrifft ein Verfahren zur Herstellung von 1-Indanolen und 1-Indanaminen durch Palladium katalysierte Arylierung sowie deren Verwendung als Zwischenprodukte für die Synthese von Feinchemikalien und von agrochemischen Wirkstoffen.

1-Indanole und 1-Indanamine der Formel (I), worin Y für eine substituierte Hydroxy- oder Aminogruppe steht, stellen ein wichtiges Strukturelement bei einer Vielzahl von agronomisch wirksamen Substanzen dar, wie sie z.B in WO 2004/069814 A1 und WO 2007/112834 A1 offenbart werden.

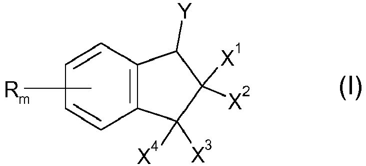

J. A. Pincock et al.: "The Photochemistry of Conformationally Rigid Benzylic Esters: 2,2-Dimethyl-1-indanyl Acetates and Pivalates", The Journal of Organic Chemistry 1995, 13, 4067, beschreiben die Herstellung von 1-Indanolen durch Reduktion der korrespondierenden 1-Indanone.
Hui Fang et al.: "Rapid Catalyst Screening by a Continuous-Flow Microreactor Interfaced with Ultra-High-Pressure Liquid Chromatography", The Journal of Organic Chemistry 2010, 16, 5619, beschreiben die Synthese eines Indanamines ausgehend vom entsprechenden 3-(3,4-Dimethoxyphenyl)-2,2-dimethylpropanenitril oder von einer Acylaminalvorstufe.
Von C. Pierre, O. Baudoin, Org. Lett. 2011, 13, 1816 und N. Martin, C. Pierre, M. Davi, R. Jazzar, O. Baudoin, O. Chem.-Eur. J. 2012, 18, 4480*,* sind Palladium(0)-katalysierte Arylierungen von 2-Halogenketonen (IIa) oder 2-Halogenverbindungen (IIb) bekannt. Bei den Ausgangsverbindungen (IIa) und (IIb) handelt es sich in beiden Fällen um solche, bei denen ein Kohlenstoffatom in 2-Position zum Halogenatom am Phenylring vorliegt, das kein Wasserstoffatom trägt (C=O in Formel IIa bzw. CRY mit R, Y jeweils ungleich Wasserstoff in Formel IIb):

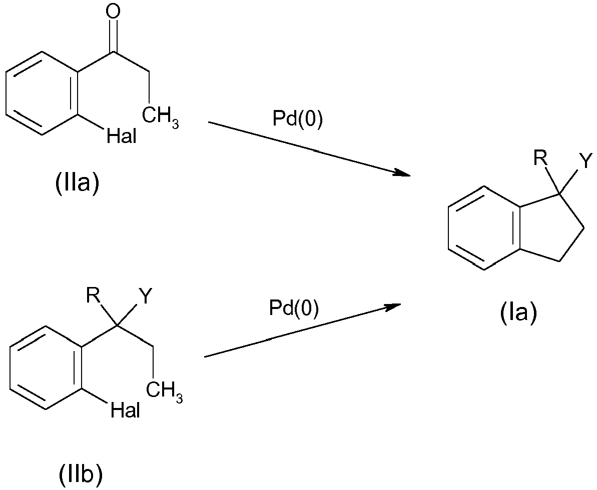

Die in diesen Schriften genannten Verfahren zur Herstellung von 1-Indanolen und 1-Indanaminen sind jedoch auf eine Durchführung im Labormaßstab beschränkt, da sie eine Reihe von Nachteilen aufweisen und somit nicht für eine industrielle Produktion anwendbar sind. Ein weiterer ganz gravierender Nachteil der aus dem Stand der Technik via Palladium katalysierter Arylierung von 2-Halogenverbindungen bekannten Verfahren ist die Tatsache, dass diese bislang ausschliesslich für solche Ausgangsverbindungen (IIa) und (IIb) beschrieben sind, bei denen ein Kohlenstoffatom in 2-Position zum Halogenatom am Phenylring vorliegt, das kein Wasserstoffatom trägt, und somit zur Bildung von 1-Indanolen und 1-Indanaminen (Ia) führen, worin R nicht Wasserstoff bedeutet.

Für die Herstellung der oben genannten agronomisch wirksamen Substanzen sind jedoch 1-Indanole und 1-Indanamine der Formel (I) als Zwischenprodukte von Interesse, d.h. solche, in denen das durch den Rest Y substituierte Kohlenstoffatom auch noch eine Wasserstoffatom trägt.

Aufgabe der vorliegenden Erfindung ist die Bereitstellung eines Verfahrens zur Herstellung von 1-Indanolen und 1-Indanaminen, welches die Nachteile der aus dem Stand der Technik bekannten Verfahren überwindet.

Es wurde nun gefunden, dass sich 1-Indanole und 1-Indanamine in hohen Ausbeuten herstellen lassen durch Palladium katalysierte Arylierung von 1-(2-Halogenphenyl)alkan-1-olen oder 1-(2-Halogenphenyl)alkan-1-aminen.

Ein Gegenstand vorliegender Erfindung ist somit ein Verfahren zur Herstellung von 1-Indanole und 1-Indanamine der allgemeinen Formel (I), dadurch gekennzeichnet, dass 1-(2-Halogen-phenyl)alkan-1-ole oder 1-(2-Halogenphenyl)alkan-1-amine (II) Palladium katalysiert in Gegenwart von Phosphin-Liganden, einer Base und eines Lösungsmittels bei erhöhter Temperatur umgesetzt werden, 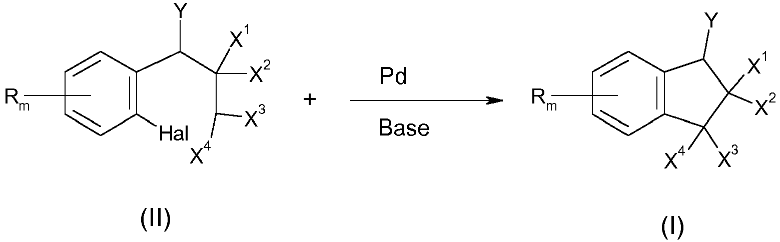 und worin die Reste, Symbole und Indices wie nachfolgend definiert sind:
- Y: bedeutet NR¹R² oder OR³,
- R¹: bedeutet Wasserstoff oder COR⁴,
- R²: bedeutet COR⁴,
oder R¹ und R² bilden gemeinsam die Gruppe COCH₂CH₂CO oder CO-Phenylen-CO,
- R³: bedeutet Si(R⁵)₃ oder Pivaloyl (2,2-dimethylpropanoyl),
- R⁴: bedeutet (C₁-C₆)-Alkyl oder Phenyl,
- R⁵: bedeutet (C₁-C₆)-Alkyl oder Phenyl,
- Hal: bedeutet Chlor, Brom oder Iod,
- R: bedeutet Fluor, (C₁-C₃)-Alkyl oder (C₁-C₃)-Alkoxy,
- X¹: bedeutet Wasserstoff oder (C₁-C₆)-Alkyl,
- X²: bedeutet Wasserstoff oder (C₁-C₆)-Alkyl,
- X³: bedeutet Wasserstoff oder (C₁-C₆)-Alkyl,
- X⁴: bedeutet Wasserstoff oder (C₁-C₆)-Alkyl,
oder
X¹ und X², oder
X³ und X⁴, oder
X¹ und X³ bilden gemeinsam mit dem Kohlenstoffatom, an das sie gebunden sind, jeweils einen 3- bis 7-gliedrigen gesättigten Ring,
oder
- Y und X¹: bilden gemeinsam mit den Kohlenstoffatomen, an die sie gebunden sind, einen 5- bis 7-gliedrigen gesättigten Ring,

- m: bedeutet 0, 1, 2 oder 3.

Geeignete Palladiumkatalysatoren sind beispielsweise Pd(OAc)2, Pd2dba3 (Tris(dibenzylidenaceton)dipalladium(0)), PdCl2 und PdBr2, wobei Pd(OAc)2 bevorzugt ist. Der Palladiumkatalysator wird üblicherweise in einer Menge von 0,1 bis 10mol%, vorzugsweise 1 bis 10 mol%, besonders bevorzugt 1 bis 5 mol%, bezogen auf die Verbindung (II) eingesetzt.

Geeignete Phosphin-Liganden sind beispielsweise Trialkylphosphine, Tricycloalkylphosphine wie Tricyclohexylphosphin, Triarylphosphine wie Triphenylphosphin, Tri-ortho-tolyolphosphin, Tri-(4-dimethylaminophenyl)-phosphine, Alkyldiarylphosphine wie Butyldiphenylphosphin, wobei Triphenylphosphin besonders bevorzugt ist. Die Phosphine können auch in Form von Salzen z.B. mit HBF₄ verwendet werden. Die Phosphin-Liganden werden üblicherweise in einer Menge von 0,1 bis 10mol%, vorzugsweise 1 bis 10 mol%, besonders bevorzugt 1 bis 5 mol%, bezogen auf die Verbindung (II) eingesetzt.

Palladiumkatalysatoren und Phosphin-Liganden können als separate Verbindungen oder zweckmäßigerweise in Form eines vorgebildeten Komplexes verwendet werden, z.B. als Tretrakis(triphenylphosphinyl)palladium, das besonders bevorzugt ist.

Geeignete Basen sind beispielsweise Carbonate, Hydrogencarbonate, Phosphate, Alkoxide und Carboxylate von Alkalimetallen, wie Li₂CO₃, Na₂CO₃, K₂CO₃, NaHCO₃ KHCO₃ K₃PO₄, K₂HPO₄, NaOMe, NaOEt, NaOiPr, NaOtBu, KOMe, KOEt, KOiPr, KOtBu, NaOAc, KOAc, NaOPiv (Natriumpivalat), KOPiv, NaOCOPh und KOCOPh oder Mischungen von solchen Basen. Bevorzugt sind Mischungen von Carbonaten und Carboxylaten. Besonders bevorzugt ist die Mischung von K₂CO₃ und KOPiv. Die Base wird üblicherweise in einem 1- bis 5-molaren Verhältnis bezogen auf die Verbindung (II) eingesetzt.

Geeignete Lösungsmittel sind beispielsweise Aromaten wie Toluol, Xylol, Chlorbenzol und Anisol; Ether wie Dibutylether, Diphenylether und Polyglycolether; Ester wie Butylacetat und Isopropylacetat; Amide wie Dimethylacetamid, Dimethylformamid, und Dibutylformamid oder Mischungen davon.
Besonders bevorzugt sind aromatische Lösungsmittel wie Xylol.

Das erfindungsgemäße Herstellverfahren wird üblicherweise bei erhöhter Temperatur von über 100°C bis zum Siedepunkt des jeweiligen Lösungsmittels durchgeführt. Bevorzugt ist ein Bereich von 120 bis 150°C. Sofern der Siedepunkt des jeweiligen Lösungsmittels darunter liegen sollte, wird zweckmäßigerweise unter Druck gearbeitet.

Bevorzugt wird das Verfahren durchgeführt für solche Verbindungen der oben genannten Formeln, in denen die Reste, Symbole und Indices wie nachfolgend definiert sind:
- Y: bedeutet NR¹R² oder OR³,
- R¹: bedeutet Wasserstoff oder COR⁴,
- R²: bedeutet COR⁴,
- R³: bedeutet Si(R⁵)₃ oder 2,2-dimethylpropanoyl,
- R⁴: bedeutet tertiär-Butyl,
- R⁵: bedeutet iso-Propyl,
- Hal: bedeutet Brom oder Iod,
- R: bedeutet Fluor, Methyl oder Methoxy,
- X¹: bedeutet Wasserstoff oder Methyl,
- X²: bedeutet Wasserstoff oder Methyl,
- X³: bedeutet Wasserstoff oder Methyl,
- X⁴: bedeutet Wasserstoff oder Methyl,
oder
X¹ und X², oder
X³ und X⁴, oder
X¹ und X³ bilden gemeinsam mit dem Kohlenstoffatom, an das sie gebunden sind, jeweils einen 3- bis 7-gliedrigen gesättigten Ring,
oder
- Y und X¹: bilden gemeinsam mit den Kohlenstoffatomen, an die sie gebunden sind, einen 5- bis 7-gliedrigen gesättigten Ring,

- m: bedeutet 0, 1, 2 oder 3.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl.

Die nachfolgenden Beispiele erläutern die Erfindung.

Die hier verwendeten Abkürzungen bedeuten:

| | | | | | |
|---|---|---|---|---|---|
| Ac | Acetat | Cy | Cyclohexyl | Cyp | Cyclopentyl |
| Me | Methyl | Ph | Phenyl | Piv | Piavalat |
| TiPs | Triisopropylsilyl | | | | |

### Allgemeine Vorschrift:

Unter Schutzgas werden der Palladium-Katalysator (0,015 mmol, 5 mol%), gegebenfalls der Phosphin-Ligand (0,03 mmol, 10 mol%) und die Base z.B. Kalium-Pivalat (0,030 mmol, 10 mol %) und K₂CO₃ (0,3 mmol, 1,0 Äq.) vorgelegt und das Lösungsmittel (3 ml Xylol) und eine Verbindung der Formel (II) (0.3 mmol, 1.0 Äq.) zugegeben. Die Mischung wird dann für 10 Minuten bei Raumtemperatur gerührt und anschließend auf 140°C erhitzt und 16 Stunden bei dieser Temperatur gerührt. Nach Abkühlen der Reaktionsmischung auf Raumtemperatur wird filtriert, das Lösungsmittel abgezogen und der Rückstand chromatografisch gereinigt.

### Beispiel 1: Herstellung von 2,2-Dimethyl-2,3-dihydro-1H-inden-1-yl)oxy)triisopropylsilan aus [1-(2-Bromphenyl)-2,2-dimethylpropoxy](triisopropyl)silan

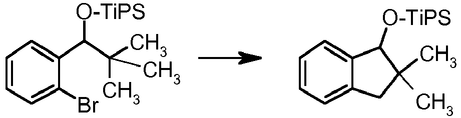

Die Reaktion erfolgte analog zur allgemeinen Vorschrift mit 5mol% Pd(PPh₃)₄ und führte zu einer Ausbeute von 89%.
¹H NMR (300 MHz, CDCl₃, 293 K) δ 1.01 (s, 3H), 1.06-1.25 (m, 24H), 2.60 (d, *J =* 15.2 Hz, 1H), 2.75 (d, *J* = 15.2 Hz, 1H), 4.93 (s, 1H), 7.11-7.22 (m, 3H), 7.29-7.38 (m, 1H).

### Beispiel 2: Herstellung von ((2',3'-Dihydrospiro[cyclopropane-1,1'-inden]-3'-yl)oxy)triisopropylsilan aus [1-(2-Bromphenyl)-cyclopropylethoxy](triisopropyl)silan

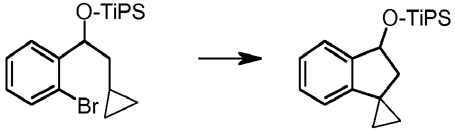

Die Reaktion erfolgte analog zur allgemeinen Vorschrift mit 5mol% Pd(PPh₃)₄ und führte zu einer Ausbeute von 69%.
¹H NMR (300 MHz, CDCl₃, 293 K) δ 0.70-0.82 (m, 1H), 0.85-0.96 (m, 1H), 1.00-1.27 (m, 23H), 2.31 (d, *J* = 7.2 Hz, 2H), 5.60 (t, *J* = 7.2 Hz, 1H), 6.67-6.76 (m, 1H), 7.17-7.27 (m, 2H), 7.39-7.46 (m, 1H).

### Beispiel 3: Herstellung von ((5,6-Dimethoxy-2,2-dimethyl-2,3-dihydro-1H-inden-1-yl)oxy)triisopropylsilan aus [1-(2-Brom-4,5-dimethoxyphenyl)-2,2-dimethylpropoxy](tri-tert-butyl)silan

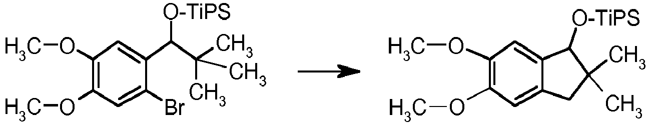

Die Reaktion erfolgte analog zur allgemeinen Vorschrift mit 5mol% Pd(PPh₃)₄ und führte zu einer Ausbeute von 84%.
¹H NMR (300 MHz, CDCl₃, 293 K) δ 1.02 (s, 3H), 1.06-1.25 (m, 24H), 2.53 (d, *J =* 15.0 Hz, 1H), 2.69 (d, *J* = 15.0 Hz, 1H), 3.85 (s, 6H), 4.86 (s, 1H), 6.69 (s, 1H), 6.89 (s, 1H).

### Beispiel 4: Herstellung von ((6-Methoxy-2,2-dimethyl-2,3-dihydro-1H-inden-1-yl)oxy)triisopropylsilan aus [1-(2-Brom-5-methoxyphenyl)-2,2-dimethylpropoxy](tri-tert-butyl)silan

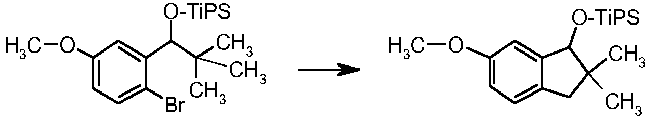

Die Reaktion erfolgte analog zur allgemeinen Vorschrift mit 5mol% Pd(PPh₃)₄ und führte zu einer Ausbeute von 81 %.
¹H NMR (300 MHz, CDCl₃, 293 K) δ 0.98 (s, 3H), 1.05-1.28 (m, 24H), 2.54 (d, *J* = 14.8 Hz, 1H), 2.65 (d, *J* = 14.8 Hz, 1H), 3.79 (s, 3H), 4.90 (s, 1H), 6.73 (dd, *J* = 2.5, 8.1 Hz, 1H), 6.89 (d, *J* = 2.5 Hz, 1H), 7.04 (d, *J* = 8.1 Hz, 1H).

### Beispiel 5: Herstellung von ((6-Fluor-2,2-dimethyl-2,3-dihydro-1H-inden-1-yl)oxy)triisopropylsilan aus [1-(2-Brom-5-fluorphenyl)-2,2-dimethylpropoxy](tri-tert-butyl)silan

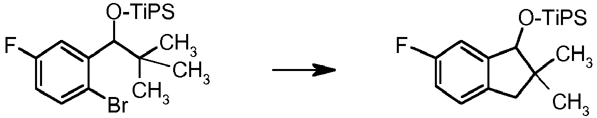

Die Reaktion erfolgte analog zur allgemeinen Vorschrift mit 5mol% Pd(PPh₃)₄ und führte zu einer Ausbeute von 84%.
¹H NMR (300 MHz, CDCl₃, 293 K) δ 1.00 (s, 3H), 1.06-1.26 (m, 24H), 2.55 (d, *J =* 15.0 Hz, 1H), 2.68 (d, *J* = 15.0 Hz, 1H), 4.90 (s, 1H), 6.87 (ddd, *J* = 2.5, 8.1, 9.2 Hz, 1H), 7.00 (dd, *J* = 2.5, 8.7 Hz, 1H), 7.07 (dd, *J* = 5.2, 8.1 Hz, 1H).

### Beispiel 6: Herstellung von 2,2-Dimethyl-2,3-dihydro-1H-inden-1-yl-2,2-dimethyl-propanoat aus 1-(2-Bromphenyl)-2,2-dimethylpropyl-2,2-dimethyl-propanoat

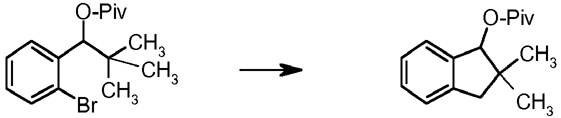

Die Reaktion erfolgte analog zur allgemeinen Vorschrift mit 5mol% Pd(PPh₃)₄ und führte zu einer Ausbeute von 95%.
¹H NMR (300 MHz, CDCl₃, 293 K) δ 1.09 (s, 3H), 1.16 (s, 3H), 1.21 (s, 9H), 2.69 (d, *J* = 15.3 Hz, 1H), 2.87 (d, *J* = 15.3 Hz, 1H), 5.81 (s, 1H), 7.09-7.29 (m, 4H).

### Beispiel 7: Herstellung von 6-Methoxy-2,2-dimethyl-2,3-dihydro-1H-inden-1-yl-2,2-dimethylpropanoat aus 1-(2-Brom-5-methoxyphenyl)-2,2-dimethylpropyl-2,2-dimethylpropanoat

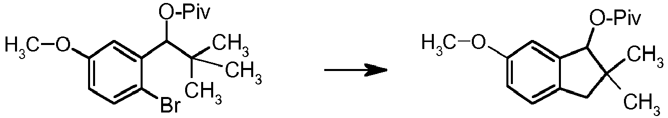

Die Reaktion erfolgte analog zur allgemeinen Vorschrift mit 5mol% Pd(PPh₃)₄ und führte zu einer Ausbeute von 88%.
¹H NMR (300 MHz, CDCl₃, 293 K) δ 1.09 (s, 3H), 1.16 (s, 3H), 1.22 (s, 9H), 2.63 (d, *J* = 15.3 Hz, 1H), 2.80 (d, *J* = 15.3 Hz, 1H), 3.77 (s, 3H), 5.79 (s, 1H), 6.77-6.84 (m, 2H), 7.05-7.12 (m, 1H).

### Beispiel 8: Herstellung von 6-Fluor-2,2-dimethyl-2,3-dihydro-1H-inden-1-yl-2,2-dimethylpropanoat aus 1-(2-Brom-5-fluorphenyl)-2,2-dimethylpropyl-2,2-dimethylpropanoat

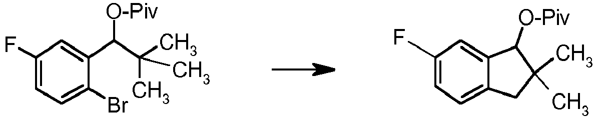

Die Reaktion erfolgte analog zur allgemeinen Vorschrift mit 5mol% Pd(PPh₃)₄ und führte zu einer Ausbeute von 76%.
¹H NMR (300 MHz, CDCl₃, 293 K) δ 1.08 (s, 3H), 1.16 (s, 3H), 1.22 (s, 9H), 2.65 (d, *J* = 15.4 Hz, 1H), 2.81 (d, *J* = 15.4 Hz, 1H), 5.76 (s, 1H), 6.87-6.99 (m, 2H), 7.08-7.15 (m, 1H).

### Beispiel 9: Herstellung von (3aR*,8bS*)-3a-methyl-3,3a,4,8b-tetrahydro-2H-indeno[1,2-b]furan-2-on aus 5-(2-Bromphenyl)-4,4-dimethyldihydrofuran-2(3H)-on

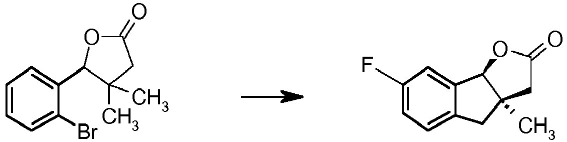

Die Reaktion erfolgte analog zur allgemeinen Vorschrift mit 5mol% Pd(PPh₃)₄ und führte zu einer Ausbeute von 88%.
¹H NMR (300 MHz, CDCl₃, 293 K) δ 1.34 (s, 3H), 2.45 (d, *J* = 17.8 Hz, 1H), 2.51 (d, *J* = 17.8 Hz, 1H), 2.87 (d, *J* = 16.4 Hz, 1H), 3.00 (d, *J* = 16.4 Hz, 1H), 5.32 (s, 1H), 7.13-7.29 (m, 3H), 7.36 (d, *J* = 7.2 Hz, 1H).

### Beispiel 10: Herstellung von 2-(2-Methyl-2,3-dihydro-1H-inden-1-yl)isoindoline-1,3-dion aus 2-[1-(2-Bromphenyl)-2-methylpropyl]-1H-isoindol-1,3(2H)-dion

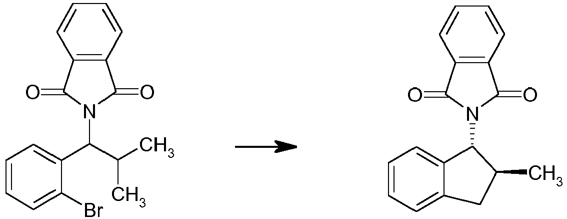

Die Reaktion erfolgte analog zur allgemeinen Vorschrift mit 5mol% Pd(PPh₃)₄ und führte zu einer Ausbeute von 54% des trans-Isomeren.
¹H NMR (300 MHz, CDCl₃, 293 K) δ 1.27 (d, *J* = 6.8 Hz, 3H), 2.65 (dd, *J* = 8.6, 15.6 Hz, 1H), 3.00-3.18 (m, 1H), 3.37 (dd, *J* = 8.2, 15.6 Hz, 1H), 5.43 (d, *J* = 8.2 Hz, 1H), 7.01 (d, *J* = 7.6 Hz, 1H), 7.11-7.19 (m, 1H), 7.20-7.30 (m, 2H), 7.70-7.79 (m, 2H), 7.82-7.91 (m, 2H).

### Beispiel 11: Herstellung von 2-(2,2-Dimethyl-2,3-dihydro-1H-inden-1-yl)isoindoline-1,3-dion aus 2-[1-(2-Bromphenyl)-2,2-dimethylpropyl]-1H-isoindol-1,3(2H)-dion

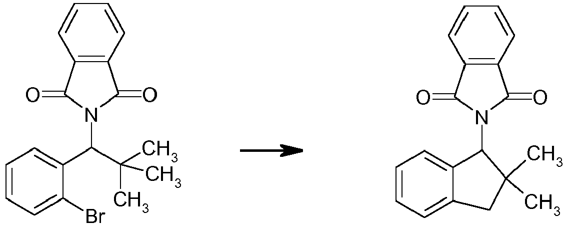

Die Reaktion erfolgte analog zur allgemeinen Vorschrift mit 5mol% Pd(PPh₃)₄ und führte zu einer Ausbeute von 86%.
¹H NMR (300 MHz, CDCl₃, 293 K) δ 0.64 (s, 3H), 0.83 (s, 3H), 2.34 (d, *J* = 15.6 Hz, 1H), 2.87 (d, *J* = 15.6 Hz, 1H), 4.9 (s, 1H), 6.66-6.77 (m, 2H), 6.80-6.89 (m, 2H), 7.16-7.33 (m, 3H), 7.42-7.49 (m, 1H).

### Beispiel 12: Herstellung von (1R,2S)-2,6-Dimethylindan-1-amin aus 2-[1-(2-Brom-5-methylphenyl)-2-methylpropyl]-1H-isoindol-1,3(2H)-dion via 2-[(1R,2S)-2,6-Dimethyl-2,3-dihydro-1H-inden-1-yl]-1H-isoindol-1,3(2H)-dion

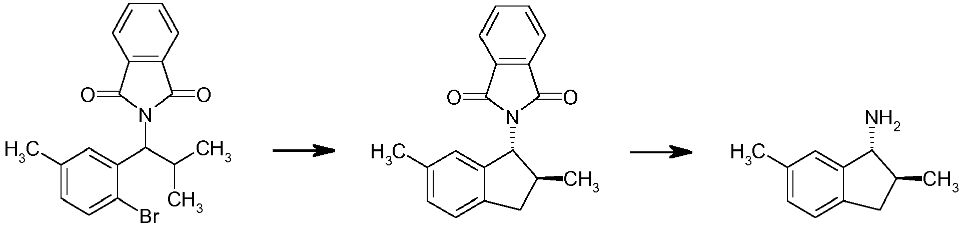

Die Reaktion erfolgte zunächst analog zur allgemeinen Vorschrift mit 5mol% Pd(PPh₃)₄ und führte zu einer Ausbeute von 55% des Primärprodukts.
¹H NMR (300 MHz, CDCl₃, 293 K) δ 1.24 (d, *J* = 7.0 Hz, 3H), 2.25 (s, 3H), 2.58 (dd, *J* = 8.0, 15.7 Hz, 1H), 2.97-3.15 (m, 1H), 3.32 (dd, *J* = 8.2, 15.7 Hz, 1H), 5.37 (d, *J* = 8.2 Hz, 1H), 6.80 (s, 1H), 7.03 (d, *J* = 7.5 Hz, 1H), 7.14 (d, *J* = 7.5 Hz, 1H), 7.70-7.78 (m, 2H), 7.80-7.90 (m, 2H).

Dann wurden zu einer Lösung von 0,17 mmol 2-(2,6-Dimethyl-2,3-dihydro-1H-inden-1-yl)isoindolin-1,3-dion in 1 ml Methanol und 1 ml THF bei 0 °C 1,7 mmol Hydrazinhydrat gegeben. Die Mischung wurde dann bei Raumtemperatur 12 Stunden gerührt. Die Mischung wurde filtriert und eingeengt. Der Rückstand wurde zwischen DCM und Wasser partitioniert, die organische Pahse abgetrennt und mit gesättigter NaHCO₃ gewaschen. Nach Trocknung über MgSO₄ und Einengung wurde das Produkt als Feststoff mit 98% Ausbeute gewonnen.
¹H NMR (300 MHz, CDCl₃, 293 K) δ 1.25 (d, *J* = 6.7 Hz, 3H), 1.68 (s, 2H), 1.90-2.08 (m, 1H), 2.35 (s, 3H), 2.44 (dd, *J* = 9.5, 15.3 Hz, 1H), 2.99 (dd, *J* = 7.7, 15.3 Hz, 1H), 3.76 (d, *J* = 8.3 Hz, 1H), 7.01 (d, *J* = 7.5 Hz, 1H), 7.08 (d, *J* = 7.5 Hz, 1H), 7.13 (s, 1H).

### Beispiel 13: Herstellung von Triisopropyl((2-methyl-2,3-dihydro-1H-inden-1-yl)oxy)silan aus [1-(2-Bromphenyl)-2-methylpropoxy](triisopropyl)silan

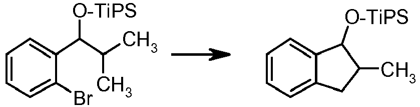

Diese Reaktion wurde mit verschiedenen Palladium-Katalysatoren, Phosphin-Liganden und Mengen an Basen durchgeführt. Die dabei unter verschiedenen Bedingungen erzielten Ergebnisse sind tabellarisch aufgeführt:

| Nr. | Pd-Kat. | Phosphin | K₂CO₃ | KOPiv | Ausbeute der Theorie |
|---|---|---|---|---|---|
| 13a | 5mol% Pd(PPh₃)₄ | | 100 mol % | 10 mol % | 55% cis, 8% trans |
| 13b | 5mol% Pd(OAc)₂ | 10 mol% PCy₃ | 110 mol % | 10 mol % | 47% cis, 5% trans |
| 13c | 5mol% Pd(OAc)₂ | 10 mol% PCyp₃ | 110 mol % | 10 mol % | 41% cis, 5% trans |
| 13d | 5mol% Pd(OAc)₂ | 10 mol% PPh₃ | 100 mol % | 10 mol % | 57% cis, 6% trans |
| 13e | 5mol% Pd(OAc)₂ | 10 mol% P(4-Me₂N-Ph)₃ | 100 mol % | 10 mol % | 48% cis, 9% trans |

cis-Isomer: ¹H NMR (300 MHz, CDCl₃, 293 K) δ 0.99 (d, *J* = 6.9 Hz, 3H), 1.08-1.25 (m, 21H), 2.56-2.68 (m, 2H), 2.92 (dd, *J* = 7.1, 15.7 Hz, 1H), 5.29 (d, *J* = 5.8 Hz, 1H), 7.17-7.25 (m, 3H), 7.35-7.43 (m, 1H).
trans-Isomer: ¹H NMR (300 MHz, CDCl₃, 293 K) δ 1.05-1.31 (m, 24H), 2.34-2.57 (m, 2H), 3.10-3.33 (m, 1H), 4.98 (d, *J* = 5.0 Hz, 1H), 7.19-7.28 (m, 3H), 7.37-7.46 (m, 1H).

## Patentansprüche

1. Verfahren zur Herstellung von 1-Indanolen und 1-Indanaminen der allgemeinen Formel (I), **dadurch gekennzeichnet, dass** 1-(2-Halogen-phenyl)alkan-1-ole oder 1-(2-Halogenphenyl)alkan-1-amine (II) Palladium katalysiert in Gegenwart von Phosphin-Liganden, einer Base und eines Lösungsmittels bei erhöhter Temperatur umgesetzt werden, 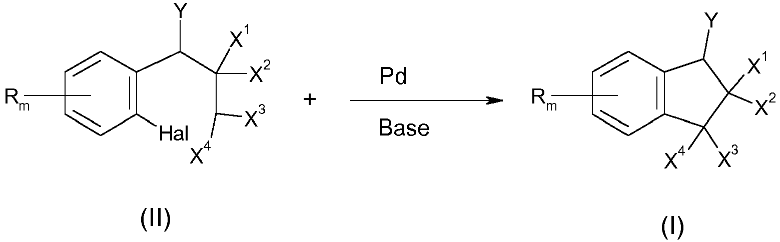 und worin die Reste, Symbole und Indices wie nachfolgend definiert sind:
Y bedeutet NR¹R² oder OR³,
R¹ bedeutet Wasserstoff oder COR⁴,
R² bedeutet COR⁴,
oder R¹ und R² bilden gemeinsam die Gruppe COCH₂CH₂CO oder CO-Phenylen-CO,
R³ bedeutet Si(R⁵)₃ oder 2,2-dimethylpropanoyl,
R⁴ bedeutet (C₁-C₆)-Alkyl oder Phenyl,
R⁵ bedeutet (C₁-C₆)-Alkyl oder Phenyl,
Hal bedeutet Chlor, Brom oder Iod,
R bedeutet Fluor, (C₁-C₃)-Alkyl oder (C₁-C₃)-Alkoxy,
X¹ bedeutet Wasserstoff oder (C₁-C₆)-Alkyl,
X² bedeutet Wasserstoff oder (C₁-C₆)-Alkyl,
X³ bedeutet Wasserstoff oder (C₁-C₆)-Alkyl,
X⁴ bedeutet Wasserstoff oder (C₁-C₆)-Alkyl,
oder
X¹ und X², oder
X³ und X⁴, oder
X¹ und X³ bilden gemeinsam mit dem Kohlenstoffatom, an das sie gebunden sind, jeweils einen 3- bis 7-gliedrigen gesättigten Ring,
oder
Y und X¹ bilden gemeinsam mit den Kohlenstoffatomen, an die sie gebunden sind, einen 5- bis 7-gliedrigen gesättigten Ring,
m bedeutet 0, 1, 2 oder 3.

2. Verfahren nach Anspruch 1, worin die Reste, Symbole und Indices folgende Bedeutungen haben:
Y bedeutet NR¹R² oder OR³,
R¹ bedeutet Wasserstoff oder COR⁴,
R² bedeutet COR⁴,
R³ bedeutet Si(R⁵)₃ oder 2,2-dimethylpropanoyl,
R⁴ bedeutet tertiär-Butyl,
R⁵ bedeutet iso-Propyl,
Hal bedeutet Brom oder Iod,
R bedeutet Fluor, Methyl oder Methoxy,
X¹ bedeutet Wasserstoff oder Methyl,
X² bedeutet Wasserstoff oder Methyl,
X³ bedeutet Wasserstoff oder Methyl,
X⁴ bedeutet Wasserstoff oder Methyl,
oder
X¹ und X², oder
X³ und X⁴, oder
X¹ und X³ bilden gemeinsam mit dem Kohlenstoffatom, an das sie gebunden sind, jeweils einen 3- bis 7-gliedrigen gesättigten Ring,
oder
Y und X¹ bilden gemeinsam mit den Kohlenstoffatomen, an die sie gebunden sind, einen 5- bis 7-gliedrigen gesättigten Ring,
m bedeutet 0, 1, 2 oder 3.

3. Verfahren nach Anspruch 1 oder 2, worin als Palladiumkatalysator eine Verbindung aus der Gruppe bestehend aus Pd(OAc)₂, Tris(dibenzylidenaceton) dipalladium(0), PdCl₂ und PdBr₂ verwendet wird.

4. Verfahren nach Anspruch 3, worin Pd(OAc)₂ verwendet wird.

5. Verfahren nach einem der Ansprüche 1 bis 4, worin Triphenylphosphin als Ligand verwendet wird.

6. Verfahren nach einem der Ansprüche 1 bis 5, worin Tretrakis(triphenyl-phosphinyl)palladium als kombinierter Palladiumkatalysator und Ligand verwendet wird.

7. Verfahren nach einem der Ansprüche 1 bis 6, worin der Palladiumkatalysator und der Ligand beziehungsweise deren Kombination jeweils in einer Menge von 0,1 bis 10 mol%, bezogen auf die Verbindung (II), eingesetzt wird.

8. Verfahren nach Anspruch 7, worin der Palladiumkatalysator und der Ligand beziehungsweise deren Kombination jeweils in einer Menge von 1 bis 5 mol%, bezogen auf die Verbindung (II), eingesetzt wird.

9. Verfahren nach einem der Ansprüche 1 bis 8, wobei als Base eine Mischung eines Alkalimetallcarbonats und eines Alkalimetallpivalats verwendet wird.

10. Verfahren nach einem der Ansprüche 1 bis 8, wobei die Base in einem 1- bis 5-molaren Verhältnis bezogen auf die Verbindung (II) eingesetzt wird.

11. Verfahren nach einem der Ansprüche 1 bis 10, wobei als Lösungsmittel ein solches aus der Gruppe der Aromaten verwendet wird.

## Claims

1. Process for preparing 1-indanols and 1-indanamines of the general formula (I) which comprises reacting 1-(2-halophenyl)alkan-1-ols or 1-(2-halophenyl)alkan-1-amines (II) at elevated temperature under palladium catalysis in the presence of phosphine ligands, a base, and a solvent, 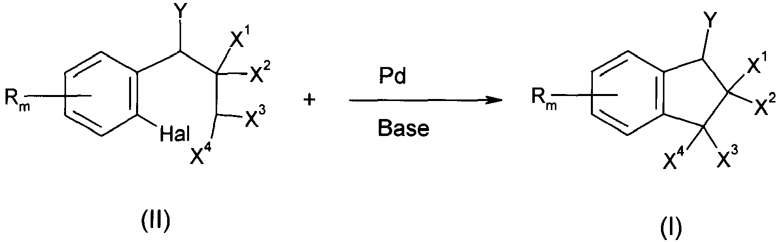 and in which the radicals, symbols, and indices are defined as follows:
Y is NR¹R² or OR³,
R¹ is hydrogen or COR⁴,
R² is COR⁴,
or R¹ and R² together form the group COCH₂CH₂CO or CO-phenylene-CO,
R³ is Si(R⁵)₃ or 2,2-dimethylpropanoyl,
R⁴ is (C₁-C₆)-alkyl or phenyl,
R⁵ is (C₁-C₆)-alkyl or phenyl,
Hal is chlorine, bromine or iodine,
R is fluorine, (C₁-C₃)-alkyl or (C₁-C₃)-alkoxy,
X¹ is hydrogen or (C₁-C₆)-alkyl,
X² is hydrogen or (C₁-C₆)-alkyl,
X³ is hydrogen or (C₁-C₆)-alkyl,
X⁴ is hydrogen or (C₁-C₆)-alkyl,
or
X¹ and X², or
X³ and X⁴, or
X¹ and X³ together with the carbon atom to which they are bonded form in each case a 3- to 7-membered saturated ring,
or
Y and X¹ together with the carbon atoms to which they are bonded form a 5- to 7-membered saturated ring,
m is 0, 1, 2 or 3.

2. Process according to Claim 1, in which the radicals, symbols, and indices have the following definitions:
Y is NR¹R² or OR³,
R¹ is hydrogen or COR⁴,
R² is COR⁴,
R³ is Si(R⁵)₃ or 2,2-dimethylpropanoyl,
R⁴ is tert-butyl,
R⁵ isisopropyl,
Hal is bromine or iodine,
R is fluorine, methyl or methoxy,
X¹ is hydrogen or methyl,
X² is hydrogen or methyl,
X³ is hydrogen or methyl,
X⁴ is hydrogen or methyl,
or
X¹ and X², or
X³ and X⁴, or
X¹ and X³ together with the carbon atom to which they are bonded form in each case a 3- to 7-membered saturated ring,
or
Y and X¹ together with the carbon atoms to which they are bonded form a 5- to 7-membered saturated ring,
m is 0, 1, 2 or 3.

3. Process according to Claim 1 or 2, in which said palladium catalyst used is a compound from the group consisting of Pd(OAc)₂, tris(dibenzylideneacetone)dipalladium(0), PdCl₂, and PdBr₂.

4. Process according to Claim 3, in which Pd(OAc)₂ is used.

5. Process according to any of Claims 1 to 4, in which triphenylphosphine is used as ligand.

6. Process according to any of Claims 1 to 5, in which tetrakis(triphenylphosphinyl)palladium is used as combined palladium catalyst and ligand.

7. Process according to any of Claims 1 to 6, in which the palladium catalyst and the ligand or the combination thereof are/is used in each case in an amount of 0.1 to 10 mol%, based on the compound (II).

8. Process according to Claim 7, in which the palladium catalyst and the ligand or the combination thereof are/is used in each case in an amount of 1 to 5 mol%, based on the compound (II).

9. Process according to any of Claims 1 to 8, where said base used is a mixture of an alkali metal carbonate and an alkali metal pivalate.

10. Process according to any of Claims 1 to 8, where the base is used in a 1- to 5-molar proportion, based on the compound (II).

11. Process according to any of Claims 1 to 10, where said solvent used is from the group of the aromatics.

## Revendications

1. Procédé de fabrication de 1-indanols et de 1-indanamines de formule générale (I), **caractérisé en ce que** des 1-(2-halogénophényl)alcan-1-ols ou des 1-(2-halogénophényl)alcan-1-amines (II) sont mis en réaction sous catalyse de palladium en présence de ligands phosphine, d'une base et d'un solvant à température élevée, 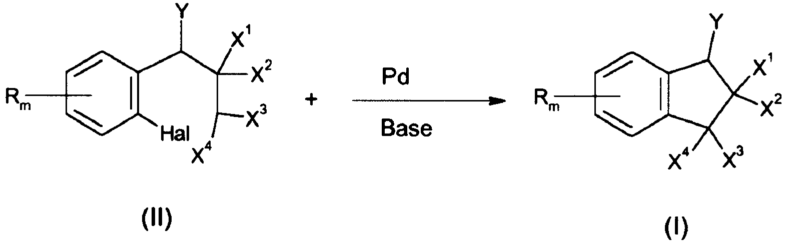 les radicaux, les symboles et les indices étant définis de la manière suivants :
Y signifie NR¹R² ou OR³,
R¹ signifie hydrogène ou COR⁴,
R² signifie COR⁴,
ou R¹ et R² forment ensemble le groupe COCH₂CH₂CO ou CO-phénylène-CO,
R³ signifie Si(R⁵)₃ ou 2,2-diméthylpropanoyle,
R⁴ signifie alkyle en (C₁-C₆) ou phényle,
R⁵ signifie alkyle en (C₁-C₆) ou phényle,
Hal signifie chlore, brome ou iode,
R signifie fluor, alkyle en (C₁-C₃) ou alcoxy en (C₁-C₃),
X¹ signifie hydrogène ou alkyle en (C₁-C₆),
X² signifie hydrogène ou alkyle en (C₁-C₆),
X³ signifie hydrogène ou alkyle en (C₁-C₆),
X⁴ signifie hydrogène ou alkyle en (C₁-C₆),
ou
X¹ et X², ou
X³ et X⁴, ou
X¹ et X³ forment chacun ensemble avec l'atome de carbone auquel ils sont reliés un cycle saturé de 3 à 7 chaînons,
ou
Y et X¹ forment ensemble avec les atomes de carbone auxquels ils sont reliés un cycle saturé de 5 à 7 chaînons,
m signifie 0, 1, 2 ou 3.

2. Procédé selon la revendication 1, dans lequel les radicaux, les symboles et les indices ont les significations suivantes :
Y signifie NR¹R² ou OR³,
R¹ signifie hydrogène ou COR⁴,
R² signifie COR⁹,
R³ signifie Si(R⁵)₃ ou 2,2-diméthylpropanoyle,
R⁴ signifie tert.-butyle,
R⁵ signifie iso-propyle,
Hal signifie brome ou iode,
R signifie fluor, méthyle ou méthoxy,
X¹ signifie hydrogène ou méthyle,
X² signifie hydrogène ou méthyle,
X³ signifie hydrogène ou méthyle,
X⁴ signifie hydrogène ou méthyle,
ou
X¹ et X², ou
X³ et X⁴, ou
X¹ et X³ forment chacun ensemble avec l'atome de carbone auquel ils sont reliés un cycle saturé de 3 à 7 chaînons,
ou
Y et X¹ forment ensemble avec les atomes de carbone auquel ils sont reliés un cycle saturé de 5 à 7 chaînons,
m signifie 0, 1, 2 ou 3.

3. Procédé selon la revendication 1 ou 2, dans lequel un composé du groupe constitué par Pd(OAc)₂, tris(dibenzylidène-acétone)-dipalladium (0), PdCl₂ et PdBr₂ est utilisé en tant que catalyseur de palladium.

4. Procédé selon la revendication 3, dans lequel Pd(OAc)₂ est utilisé.

5. Procédé selon l'une quelconque des revendications 1 à 4, dans lequel la triphénylphosphine est utilisée en tant que ligand.

6. Procédé selon l'une quelconque des revendications 1 à 5, dans lequel le tétrakis(triphénylphosphinyl)-palladium est utilisé en tant que catalyseur de palladium et ligand combinés.

7. Procédé selon l'une quelconque des revendications 1 à 6, dans lequel le catalyseur de palladium et le ligand ou leur combinaison sont utilisés respectivement en une quantité de 0,1 à 10 % en moles, par rapport au composé (II).

8. Procédé selon la revendication 7, dans lequel le catalyseur de palladium et le ligand ou leur combinaison sont utilisés respectivement en une quantité de 1 à 5 % en moles, par rapport au composé (II).

9. Procédé selon l'une quelconque des revendications 1 à 8, dans lequel un mélange d'un carbonate de métal alcalin et d'un pivalate de métal alcalin est utilisé en tant que base.

10. Procédé selon l'une quelconque des revendications 1 à 8, dans lequel la base est utilisée en un rapport molaire de 1 à 5 par rapport au composé (II).

11. Procédé selon l'une quelconque des revendications 1 à 10, dans lequel un solvant du groupe des composés aromatiques est utilisé en tant que solvant.
